# EUROPEAN PATENT APPLICATION

(11) **EP 3 167 895 A1**
(43) Date of publication of application: **17.05.2017**
(21) Application number: 15193832.1
(22) Date of filing: 10.11.2015
(51) Int. Cl.: A61K 38/01, A61K 31/716, A23K 50/60, A23K 50/30, A23K 10/16, A23K 10/20, A23L 29/269, A23L 29/281, A23L 33/00, A23L 33/14, A23L 33/18, A23L 33/19, A23L 33/21

(54) **A DIETARY SUPPLEMENT COMPRISING BETA-GLUCAN AND CASEIN HYDROLYSATE FOR IMPROVING HEALTH AND GROWTH PERFORMANCE IN A MAMMAL**

(71) Applicant: UNIVERSITY COLLEGE DUBLIN, Dublin 4 (IE)
(72) Inventor: SWEENEY, Torres, Co. Kildare (IE); O'DOHERTY, John, Co. Kildare (IE)
(74) Representative: Purdy, Hugh Barry

(57) **Abstract**

A non-therapeutic method of improving growth performance of a weaning mammal comprises a step of administering to the weaning mammal or a maternal mammal an effective amount of beta-glucan and casein hydrolysate, wherein the weight ratio of beta-glucan to casein hydrolysate is 1:2 to 2:1. A dietary supplement suitable for oral administration to a mammal comprising beta-glucan and casein hydrolysate is also provided, wherein the weight ratio of beta-glucan to casein hydrolysate is 1:2 to 2:1.

## Description

### Introduction

The invention relates to a composition for improving health and growth performance in a mammal especially a weaning mammal. In particular, the invention relates to a composition for improving growth performance in a weaning mammal.

### Background to the Invention

Post-weaning complications in piglets are characterized by heightened susceptibility to infection, reduction in body weight, diarrhea and atrophy of small intestine structure (Burrin, 2003; Lalles et al., 2007). After the ban on prophylactic antibiotics and growth promoters, zinc oxide (ZnO) has been extensively used in weaning piglet diets (Sargeant et al., 2010), but only recently, excessive use of zinc in animal feed has been associated with concerns regarding environmental pollution (Sales, 2013).

In previous experiments, a ZnO based dietary treatment group was associated with improved overall growth performance parameters and anti-inflammatory activity in the weaning piglet gut (Heim et al., 2014). Due to the rise in environmental pollution due to the use of zinc in animal diet, more organic and natural feed alternatives are being explored to control weaning period complications.

It is an object of the invention to overcome at least one of the above-referenced problems.

### Statements of Invention

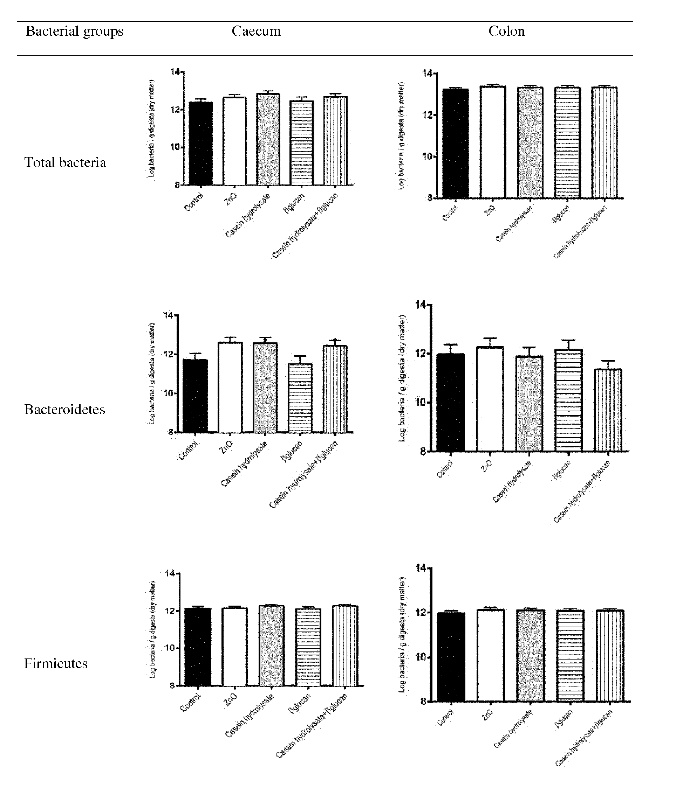

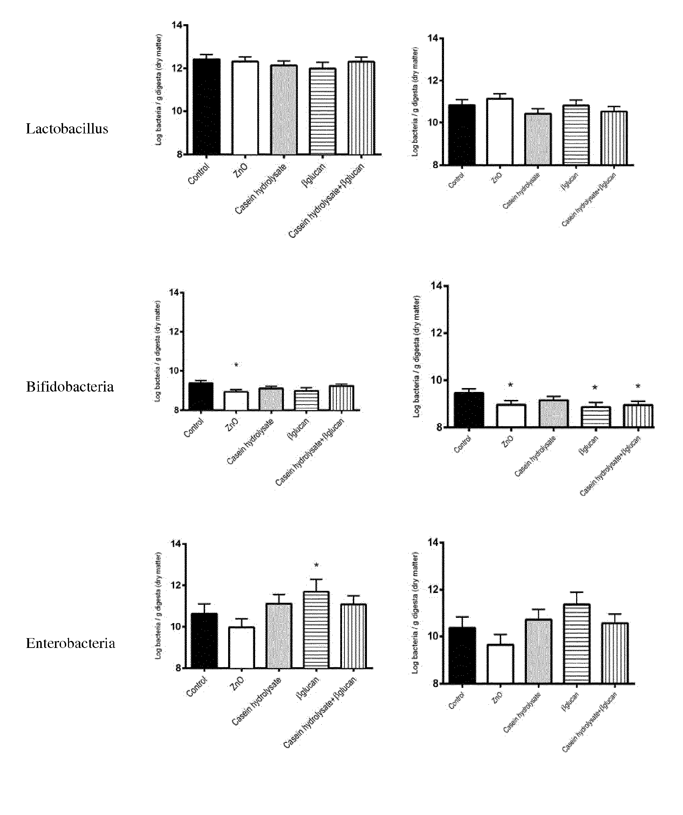

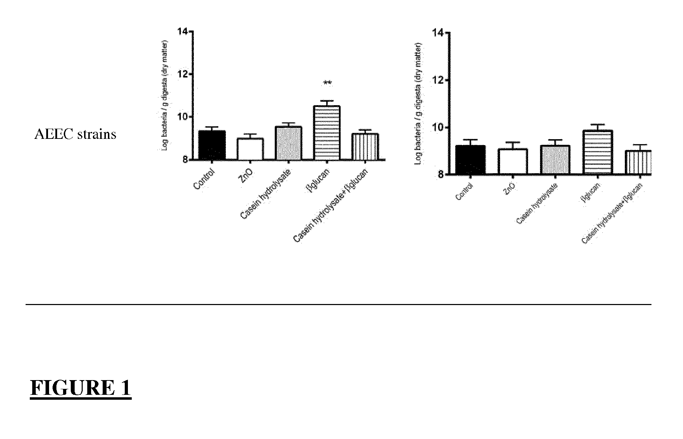

The applicant has discovered that supplementing a weaning mammals diet with a composition comprising beta-glucan and a casein hydrolysate improves growth performance of the weaning animal over a 12-day period. In particular, administration of beta-glucan and a casein hydrolysate to a weaning mammal resulted in lower faecal scores compared to a control diet (Table 2), increase in overall average daily gain compared to a control diet (Table 3), increase in feed intake compared to a control diet (Table 3), and increase in gain to feed ratio compared to a control diet (Table 3). There was also a diminished abundance of eneteropathogenic AEEC strains compared with a beta-glucan diet (Fig.1) and a decrease in expression of the cytokine IL-1.

In one aspect, the invention provides a composition comprising (or consisting essentially of) beta glucan and casein hydrolysate, typically in a weight ratio of 1:3 to 3:1. In one embodiment, the weight ratio is 1:2 to 2:1.

In one embodiment, the beta-glucan is yeast-derived beta-glucan.

In one embodiment, the casein hydrolysate is depleted in high molecular weight peptides.

In one embodiment, the casein hydrolysate has a degree of hydrolysis of 5-15%DH.

In one embodiment, the composition comprises 10 to 90% beta-glucan (w/w) and 10 to 90% casein hydrolysate (w/w).

In one embodiment, the composition comprises 30 to 70% beta-glucan (w/w) and 30 to 70% casein hydrolysate (w/w).

In one embodiment, the composition comprises 40 to 60% beta-glucan (w/w) and 40 to 60% casein hydrolysate (w/w).

In one embodiment, the composition is provided in the form of a particulate product, for example powder, flakes, pellets, crumbles or granules. In one embodimet, the composition is liquid.

In one embodiment, the composition is provided in the form of a dietary supplement for a mammal.

In a further aspect, the invention provides a food product for animals comprising a composition according to the invention. In one embodiment, the composition comprises 0.001 to 1.0% of the food product (w/w). In one embodiment, the composition comprises 0.005 to 0.5% of the food product (w/w). In one embodiment, the composition comprises 0.005 to 0.1% of the food product (w/w). In one embodiment, the composition comprises 0.01 to 0.03% of the food product (w/w). In one embodiment, the composition comprises about 0.02% of the food product (w/w). In one embodiment, the food product is a human food product. In one embodiment, the food product is an animal feed. In one embdoiment, the animal is a weaning animal. In one embodiment, the animal is a post-weaning mammal. In one embodiment, the animal is a pregnant animal. In one embodiment, the animal is a lactating animal.

In one embodiment, the animal feed is formulated for oral administration to a weaning mammal. In one embodiment, the animal feed is formulated for oral administration to a maternal mammal as part of their diet.

In one embodiment, the animal feed is formulated for a weaning piglet, weaning calf or weaning lamb.

In a further aspect, the invention provides a composition of the invention, for use in improving health and growth performance of a mammal, in which the composition is administered to the weaning mammal.

In a further aspect, the invention provides a composition of the invention, for use in improving health and growth performance of a weaning mammal, in which the composition is administered to the weaning mammal or the maternal mammal.

In one embodiment, the composition is administed to the weaning mammal.

In one embodiment, the composition is administed to the maternal animal.

In one embodiment, the weaning mammal is a weaning pig and the maternal mammal is a maternal pig.

In one embodiment, the invention provides a composition of the invention, for use in maintaining or improving gut health in a mammal.

In another apsect, the invention provides a composition of the invention, for use in preventing or treating a gastrointestinal inflammatory condition in a mammal.

In another aspect, the invention provides a pharmaceutical composition comprising an effective amount of beta-glucan and casein hydrolysate in combination with a pharmaceutically acceptable excipient.

In another aspect, the invention provides a method (typically non-therapeutic method) of improving health and growth performance of a mammal comprising a step of administering to the mammal an effective amount of beta-glucan and casein hydrolysate.

In one embodiment, the invention provides a method (typically non-therapeutic method) of improving health and growth performance of a weaning mammal comprising a step of administering to the weaning mammal or the maternal mammal an effective amount of beta-glucan and casein hydrolysate.

In one embodiment, the method of the invention comprises administering a composition of the invention to the mammal, weaning mammal, or maternal mammal.

In one embodiment, the method of the invention comprises administering a composition of the invention to the maternal mammal during pregnancy, lactation, or both pregnancy and lactation.

In one embodiment, the method of the invention comprises administering a composition of the invention to the mammal prior to and after weaning.

In one embodiment, the weight ratio of beta-glucan to casein hydrolysate administered to the mammal is 1:3 to 3:1.

In one embodiment, the weight ratio of beta-glucan to casein hydrolysate administered to the mammal is 1:2 to 2:1.

In one embodiment, the weight ratio of beta-glucan to casein hydrolysate administered to the mammal is 2:3 to 3:2.

In one embodiment, the weight ratio of beta-glucan to casein hydrolysate administered to the mammal is about 1:1.

In one embodiment, the method comprises administering to the mammal 1-1000 mg beta-glucan per day per Kg body weight and 1-1000 mg casein hydrolysate per day per Kg body weight.

In one embodiment, the method comprises administering to the mammal 1-500 mg beta-glucan per day per Kg body weight and 1-500 mg casein hydrolysate per day per Kg body weight.

In one embodiment, the method comprises administering to the mammal 10-100 mg beta-glucan per day per Kg body weight and 10-100 mg casein hydrolysate per day per Kg body weight.

In one embodiment, the method comprises administering to the mammal 10-50 mg beta-glucan per day per Kg body weight and 10-50 mg casein hydrolysate per day per Kg body weight.

In one embodiment, the method comprises administering to the weaning mammal 10-1000 mg beta-glucan per day per Kg body weight and 10-1000 mg casein hydrolysate per day per Kg body weight.

In one embodiment, the method comprises administering to the weaning mammal 10-100 mg beta-glucan per day per Kg body weight and 10-100 mg casein hydrolysate per day per Kg body weight.

In one embodiment, the method comprises administering to the weaning mammal 20-60 mg beta-glucan per day per Kg body weight and 20-60 mg casein hydrolysate per day per Kg body weight.

Suitably, the beta-glucan is derived from bakers yeast.

Suitably, the effective amount of beta-glucan and casein hydrolysate is administered in a plurality of doses over a period of at least 5 days.

Suitably, the weaning mammal is a piglet.

### Definitions:

"Beta-glucan" or "β -glucan" means a homopolysaccharide of linear or branched glucose monomers linked by β-glycosidic bonds. They occur in plants, cereals, seaweed and fungi (for example the cell wall of Saccharomyces cerevisiae). The term includes β(1,3)-glucan, β(1,3)D-glucan, β(1,4)-glucan, β(1,3(1,4)-glucans, and β(1,3(1,6)-glucans.. The beta-glucan may be soluble, partially soluble, insoluble beta-glucans or any mixture thereof. Preferably, the beta-glucan has GRAS food ingredient status. The β-glucans found in yeast are long linear chains of up to 1300-1500 residues of glucan molecules linked by β(1-3) bonds with a small amount of β(1-6) chains. Algal β-glucans (also known as lamarin) have smaller chain lengths (20-30 residues) with a minor amount of β(1-6) chains. Beta-glucans are commercially available from a number of sources, including Wellmune® (yeast beta-glucan), Barliv ®, barley beta glucan, and OatWell® (oat beta glucan).

"Dietary supplement" means a product that is suitable for mammalian ingestion as a means of supplementing the mammals primary diet. In one embodiment, the supplement is formulated to supplement the diet of weaning mammals, for example weaning pigs. In one embodiment, the supplement is provided is a particulate form, suitable for ingestion by the mammal or for mixing the supplement with an animal feed. Examples of particulate forms include powders, pellets, flakes, granules, and the like. Typically, the dietary supplement comprises 0.01 to 0.1% (w/w) of the weaning mammals adult (non-mothers milk) diet. Typically, the dietary supplement comprises 0.03 to 0.07% (w/w) of the weaning mammals adult (non-mothers milk) diet. Typically, the dietary supplement comprises 0.04 to 0.06% (w/w) of the weaning mammals adult (non-mothers milk) diet.

"Yeast beta-glucan" means beta-glucan obtained from yeast. In one embodiment, the yeast beta-glucan is obtained from the cell wall of *Saccharomyces cerevisciae* (Bakers Yeast).

"Casein hydrolysate" means a hydrolysate of casein or a casein derivative such as a caseinate. It is produced by hydrolysing a casein substrate to break up protein into small peptides and polypeptides. Hydrolysis can be thermal or enzymatic hydrolysis. In one embodiment, the hydrolysate is produced by proteolytic hyrdolysis using a suitable proteolytic enzyme or enzyme preparation, for example a subtilisin protease preparation. Examples of subtilisin protease preparations include Alkalase, ALK_enzyme, Bioprase, Genenase I, Protamex, Thermoase, Superase and Savinase. In one embodiment, the protease is derived from a fungus. In one embodiment, the protease is derived from a bacteria. In one embodiment, the protease is derived from an aspergillus fungus. In one embodiment, the protease is derived from a Bacillus species of bacteria. In one embodiment, the casein hydrolysate has a degree of hydrolysis (%DH) of 5-50. In one embodiment, the casein hydrolysate has a degree of hydrolysis (%DH) of 5-40. In one embodiment, the casein hydrolysate has a degree of hydrolysis (%DH) of 5-30. In one embodiment, the casein hydrolysate has a degree of hydrolysis (%DH) of 5-20. In one embodiment, the casein hydrolysate has a degree of hydrolysis (%DH) of 5-15. A method of measuring %DH for a casein hydrolysate is described in WO2012110652. In one embodiment, the casein hydrolysate is product by hydrolysis of a caseinate (a metallic salt of casein). Examples of caseinates include sodium caseinate, calcium caseinate, potassium caseinate, and sodium/calcium caseinate. In one embodiment, the caseinate is sodium caseinate. In a preferred embodiment, the casein substrate is a milk casein, ideally a bovine milk casein. Typically, the casein substrate (and/or the casein hydrolysate) is substantially free (i.e. less than 5%, 4%, 3%, 2%, 1%, 0.5%, 0.2%, 0.1% (w/w) of other milk proteins, for example whey proteins. The casein substrate employed for the hydrolysis typically has a concentration of 5-15%, 8-12%, and ideally about 9-11% (w/v). Typically, the inclusion rate of protease (w/w) is from 0.1 to 5.0%, preferably 0.2 to 2%, more preferably 0.5 to 1.5%, and ideally at about 1%. Typically, the protease employed has an activity of 1-2 AU-N/g. Suitably, hydrolysis of the casein substrate with protease is carried out at a temperature of from 45°C to 55°C, preferably from 49°C to 51°C, and ideally at about 50°C. Typically, hydrolysis of the casein substrate with protease is carried out at a pH of from 5 to 9, suitably at a pH of from 5.5 to 8, preferably at a pH of from 6 to 8, and ideally at a pH of about 7.

"Depleted in high molecular weight peptides" as applied to the casein hydrolysate means having less than 20%, and preferably less than 10%, of peptides having a molecular weight of greater than 8KDa. In one embodiment, the casein hydrolysate comprises less than 8% of peptides having a molecular weight of greater than 8KDa. In one embodiment, the casein hydrolysate comprises less than 6% of peptides having a molecular weight of greater than 8KDa. In one embodiment, the casein hydrolysate comprises less than 4% of peptides having a molecular weight of greater than 8KDa.

"Effective amount" as applied to beta-glucan and casein hydrolysate in the context of improving growth parameters should be understood to mean an amount of both beta-glucan and casein hydrolysate that is sufficient when administered to the weaning mammal or the maternal mammal to improve the health and growth performance of the weaning animal. In one embodiment, an effective amount means at least 1mg/Kg body weight/day. In one embodiment, an effective amount means 1-1000 mg/Kg body weight/day. In one embodiment, an effective amount means 10-500 mg/Kg body weight/day. In one embodiment, an effective amount means 10-100 mg/Kg body weight/day. In one embodiment, an effective amount means 10-70 mg/Kg body weight/day. In one embodiment, an effective amount means 10-60 mg/Kg body weight/day. In one embodiment, an effective amount means 20-60 mg/Kg body weight/day. In one embodiment, the beta-glucan and casein hydrolysate is administered as a bolus dose, for example 1-100g, 1-5g, 1-10g, 10-100g, or 10-50g.

"Food for animals" means animal feed and food for humans. Food for humans includes solid and liquid foods.

"Animal feed" means a feed formulated for animal or mammals, especially domestic mammals such a pigs, sheep, cows and goats. The animal feed may be fodder, for example pelleted feeds and feed grains, or may be a slurry or liquid. The animal feed may be a compound feed, for example meal pellets or crumbles.

"Mammal" means a human or domestic mammal such as for example a cow, sheep, goat or pig. In one embodiment the mammal is a pig.

"Weaning mammal" means a young mammal that is being fed mothers milk and optionally adult feed (in the case on non-human animals) and solid food (in the case of humans), and is about to or is in the process of being "weaned off" mothers milk. Pigs are conventionally weaned at 3-5 weeks of age, and in some case 10 days to 3 weeks. Bovine calves are weaned at 7-8 months of age, and sometimes at 6 months of age. Humans are weaned at 6-12 months.

"Improving health and growth performance" means one or more of lower faecal scores compared to a control diet, increase in overall average daily gain compared to a control diet, increase in feed intake compared to a control diet, increase in gain to feed ratio compared to a control diet, and diminished abundance of eneteropathogenic AEEC strains and improved gut health. In one embodiment, the improved growth performance is comparable to that achieved with a ZnO supplementation.

"Maternal mother" means the mother of the weaning mammal. This, in one embodiment, the feed supplement of the invention is fed to the maternal mammal instead of the weaning mammal. It can be fed to the mother during pregnancy, lactation or both. In one embodiment, the feed supplement is fed to both the weaning mammal and the maternal mother.

"Improving gut health" means one or more of improved digestion and absorption of food, reduction or inhibition of GI illness, improvement in or maintenance of normal and stable intestinal microflora, improved immune status, or maintained healthy gut status. "Effective amount" as applied to beta-glucan and casein hydrolysate in the context of improving gut health should be understood to mean an amount of beta-glucan and casein hydrolysate which results in a clinically significant improvement in gut health.

"Gastrointestinal inflammatory condition" means a disease or condition that causes inflammation of at least part of the gastrointestinal tract, and includes Inflammatory Bowel Disease (IBD), Irritable Bowel Syndrome (IBS), gastritis, chronic gastrointestinal inflammation, and gastrointestinal cancer such as colon cancer. In one embodiment, the composition and methods of the invention inhibit the development of gastrointestinal inflammatory conditions. "Effective amount" as applied to beta-glucan and casein hydrolysate in the context of prevention or treatment of gastrointestinal inflammatory conditions should be understood to mean an amount of beta-glucan and casein hydrolysate which results in a clinically significant inhibition, amelioration or reversal of symptoms of gastrointestinal inflammation.

"Pharmaceutical composition" means a therapeutically effective amount of the therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, *etc.* Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

### Brief Description of the Figures

Figure 1: Effect of feed additives on copy numbers of a panel of selected bacterial groups present in caecal digesta.
Figure 2: Effect of feed additives on relative quantity (RQ) of a panel of selected cytokine genes in porcine colonic tissues.

### Detailed Description of the Invention

### Materials and methods

All procedures described in this experiment were conducted under experimental license from the Irish Department of Health in accordance with the Cruelty to Animals Act 1876 and the European Communities (amendments of the Cruelty to Animals Act 1876) regulations, 1994.

### Experimental Design and Dietary Treatments

This study was designed in a completely randomized block design with 5 dietary groups. The dietary groups are as follows: 1) control diet, 2) control diet + ZnO, 3) control diet + casein hydrolysate, 4) control diet + β glucan and 5) control diet + casein hydrolysate + β glucan. The control diet used in this study was based on previous work by Walsh et al. (2013). The levels of casein hydrolysate used were based on previous experiment and the levels of β glucan, derived from yeast (*S. cerevisiae*), used were based on a previous study by Sweeney et al. (2012). Diets were formulated to contain similar concentrations of digestible energy (DE) (14.5 MJ/kg) and standardized ileal digestible (SID) lysine (12.5 g/kg) contents. All amino acids requirements were met relative to SID lysine (NRC, 1998). All diets were milled on site and fed in meal form for 12 days after weaning. The composition and analysis of experimental diets is presented in Table 1.

**Table 1: Composition of dietary treatments.**

| **Ingredient (g/kg)** | **Control** | **ZnO** | casein hydrolysate | **β glucan** | casein hydrolysate + **β glucan** |
|---|---|---|---|---|---|
| Whey powder | 50 | 50 | 50 | 50 | 50 |
| Wheat | 380 | 380 | 380 | 380 | 380 |
| Barley | 233.5 | 233.5 | 233.5 | 233.5 | 233.5 |
| Soya bean meal | 170 | 170 | 170 | 170 | 170 |
| Full fat soybean | 120 | 120 | 120 | 120 | 120 |
| Soya oil | 10 | 10 | 10 | 10 | 10 |
| Vitamins and minerals | 3 | 3 | 3 | 3 | 3 |
| Salt | 3 | 3 | 3 | 3 | 3 |
| Dicalcium phosphate | 12.5 | 12.5 | 12.5 | 12.5 | 12.5 |
| Limestone | 11 | 11 | 11 | 11 | 11 |
| Lysine HCL | 4 | 4 | 4 | 4 | 4 |
| DL-methionine | 1.5 | 1.5 | 1.5 | 1.5 | 1.5 |
| L-threonine | 1.5 | 1.5 | 1.5 | 1.5 | 1.5 |
| Zinc Oxide | | 3.2 | | | |
| Casein hydrolysate | | | 0.25 | | 0.25 |
| β glucan | | | | 0.25 | 0.25 |

### Animals and Management

Forty weaned piglets [progeny of Landrace boars x (Large White x Landrace sow)] were weighed and blocked on the basis of initial live body weight (7.3 ± 0.2 kg) and assigned to 1 of the 5 dietary treatment groups (n = 8). Piglets were weighted individually at the beginning of the experiment (day 0 = day of weaning), day 6 and day 12. The piglets were housed in groups of two on fully slatted floors (1.68 × 1.22 m). The ambient environmental temperature within the house was thermostatically controlled at 30°C. The feed was available *ad libitatum* and water was available via nipple drinkers. Feed was available up to the final weighing and all remaining feed was weighed back for the purpose of calculating feed efficiency.

### Feces scoring

From day 0 of the experiment until day 12, fecal scores were assigned daily to individual pens. Each pen floor was observed daily and the feces were scored based on their consistency. The following scoring system was used to assign fecal scores: 1 = hard feces, 2 = slightly soft feces, 3 = soft, partially formed feces, 4 = loose, semi-liquid feces, 5 = watery, mucous-like feces. This scoring system was used to indicate the presence and severity of diarrhea, as described in a previous study by Pierce *et al.* (2005).

### Animal sacrifice and sample collection

At day 12, all the pigs were sacrificed following a lethal injection of Euthatal (pentobarbitone sodium BP- Merial Animal Ltd, Sandringham House, Essex, UK) at a rate of 1 ml/1·4 kg live body weight. In sterile conditions, the digestive tract was removed and tissue samples from colon were placed in sterile phosphate buffer saline (PBS) (Oxoid, Basingstoke, UK). The digesta samples were recovered aseptically from caecum and colon and transported on ice to proper storage conditions.

### Extraction of microbial DNA from caecal and colonic digesta

To evaluate the effect of feed additives on the microbiota, samples of digesta contents (approximately 10 g) were recovered from the caecum and colon of each animal in sterile conditions immediately after slaughter. Digesta samples were stored in sterile containers (Sarstedt, Wexford, Ireland), placed on ice and transported to the laboratory within 2 h. Bacterial genomic DNA was extracted from digesta samples using a QIAamp DNA stool kit (Qiagen, West Sussex, UK) following manufacturer's instructions. Quantity and quality of DNA were assessed using a Nanodrop (Nanodrop, ND1000; Thermo Scientific, Wilmington, DE).

### Preparation of standard curves

A combined aliquot of bacterial genomic DNA from all animals were used for preparation of standard curves. Phylum, species and genus specific primers were selected to amplify a segment of the gene encoding 16s rRNA using bacterial DNA from the pooled DNA using PCR. Thermal cycling conditions used were similar to the condition described below for quantitative real-time PCR. Standard curves were subsequently generated by quantitative real-time PCR of serial dilutions of these amplicons using the same genus and species-specific primers to permit absolute quantification based on gene copy number (Lee et al., 2006). Estimation of selected bacterial groups in cecal and colonic digesta were performed using quantitative real-time PCR (ABI 7500 Fast Real-Time PCR System; Applied Biosystems Ltd., Warrington, UK). For bacterial groups, real-time PCR were performed in a final reaction volume of 20 µL containing 1 µL template DNA, 1 µL of forward and reverse primers (100 pM), 10 µL fast SYBR Green PCR Master Mix (Applied Biosystems Ltd.) and 8 µL nuclease-free water. The thermal cycling conditions involved an initial denaturation step at 95°C for 10 min followed by 40 cycles of 95°C for 15 s and 65°C for 1 min. Dissociation analyses of the PCR product were performed to confirm the specificity of the resulting PCR products. The mean threshold cycle values from the triplicate of each sample were used for calculations. The dry matter (DM) of the digesta was determined after drying overnight at 103°C.

Estimates of gene copy numbers for select bacteria were log transformed and are presented as gene copy numbers per gram of DM of digesta.

### RNA extraction

Tissue samples were collected from the mesenteric side of the colon, rinsed with ice-cold sterile phosphate-buffered saline (Oxoid, Basingstoke, UK) and stripped of overlying smooth muscle. The samples were then cut into small pieces using a sterile scalpel blade and stored in 15 ml of RNAlater™ (Applied biosystems, Foster city, CA, USA) overnight followed by storage at -20°C prior to RNA extraction. Total RNA was extracted from the colonic tissue samples using a GenElute Mammalian Total RNA Miniprep Kit (Sigma-Aldrich Corporation, St Louis, MO, USA) according to the manufacturer's instructions. To eliminate possible genomic DNA contamination, total RNA samples were subjected to DNAse I (Sigma-Aldrich Corporation, St Louis, MO, USA) treatment according to the manufacturer's instructions followed by further purification using a phenol-chloroform extraction method. The total RNA was quantified and assessed for purity using the NanoDrop-ND1000 Spectrophotometer (Thermo Fisher Scientific Inc. MA, USA). The quality of the total RNA was determined by visualizing on an ethidium bromide stained 1% agarose gel.

### cDNA syntheses

Total RNA (1µg) was used for the synthesis of First Strand *cDNA* using the First Strand *cDNA* Synthesis Kit (Qiagen Ltd. Crawley, UK) using oligo dT primers following the manufactures instructions. After the *cDNA* synthesis the final volume adjusted to 120µl with nuclease free water.

### Quantitative Real-Time PCR (qPCR)

qPCR was carried out to quantify the following two sets of targets: the first set of target are a selected panel of cytokine genes; Interleukins (*IL-1α, IL-1β, IL-4, IL-6, IL-8, IL-10, IL-17A, IL-21*), Interferon (*IFN-γ*), Tumor Necrosis Factor (*TNFα*), Transforming growth factor (*TGF-β*) and Forkhead box P3 (*FOXP3*). The second set of primers target the members of suppressors of cytokine signalling (SOCS) family which include *SOCS1, SOCS2, SOCS3, SOCS4, SOCS5, SOCS6, SOCS7* and cytokine inducible Src Homology 2 protein (*CIS*)*.* The primer efficiency was determined using a serial dilution (1:4 dilution series over 7 points) of a *cDNA* pool, prepared by pooling an equal quantity of *cDNA* from all of the experimental samples, the efficiency of all primers was between 90% to 110%. Glyceraldehyde 3-phosphate dehydrogenase (*GAPDH*), β₂ microglobulin (*B2M*), Beta-actin (*ACTB*), Peptidylprolyl isomerase A (*PPIA*) and 14-3-3 protein zeta/delta (*YWHAZ*) were used as endogenous controls as described by Ryan et al. (2010). All primers were designed using Primer Express ™ software and were synthesised by MWG Biotech (Milton Keynes, UK). This assay was carried out using 96 well fast optical plates on a 7500HT ABI Prism Sequence Detection System (PE Applied Biosystems, Foster City, CA) using fast SYBR Green PCR Master Mix (Applied Biosystems). All reactions were performed in triplicate in a total volume of 20 µl containing 10µl SYBR PCR Master mix, forward and reverse primer (5µM) (1µl), 8µl DEPC treated water and 1µl of template cDNA. The thermal cycling conditions were as follows, 95 °C for 10 min, 40 cycles of 95°C for 15s and 65°C for 1 min. Dissociation analysis confirmed the specificity of the resulting PCR products.

### Normalization of data

Mean Ct values were converted to relative quantities using the formula, Relative quantity = (PCR efficiency) ^{ΔCt}, where ΔCₜ is the change in the Ct values of the sample relative to the highest expression (minimum Ct value). Relative quantities for the endogenous controls were input in to geNorm (Vandeseaomple, 2002), a normalization factor was obtained from the four most stable (M < 1.5) reference genes (*GAPDH, B2M, ACTB* and *PPIA*) for both the ileum and the colon. The relative quantities for the target genes were then divided by the normalization value to give the final normalized value for each target gene in each sample.

### Statistical analysis.

The current study was a complete randomised design experiment and data analysed using the general linear model procedure of the Statistical Analysis Institute (SAS, 2004). The individual pen represented the experimental unit for the performance analysis. All the data were checked initially for normality using the PROC univariate procedure in SAS (2004). The microbial counts were log transformed before statistical analysis. Probability values of less than 0.05 were used as the criterion for statistical significance. Intercorrelations between cytokines and cytokine regulatory genes was analysed in a correlation matrix using PASW 18 (SPSS for Windows, version 11.0; SPSS, Chicago, IL) and the Pearson's r value greater than 0.400 was considered to be positively correlated. All results are presented in the tables as least square means ± standard error of the means (SEM).

### Results

### Fecal scoring and weight gain over period of 12 days post weaning

The effect of dietary inclusion of ZnO, casein hydrolysate, yeast β glucan and a combination of the casein hydrolysate and β glucan on faecal scores and weight of animals is presented in Table 2. The fecal scores of piglets from ZnO, casein hydrolysate, β glucan and casein hydrolysate + β glucan dietary groups were not different compared to control diet group piglets from day 0-6. The ZnO group piglets were associated with a lower fecal scores on day 6-12 (2.0 vs 3.2 ± 0.25, P<0.01) as well as overall fecal scores (1.9 vs 2.8 ± 0.21, P<0.05) relative to control-diet group. Similarly, casein hydrolysate + β glucan group piglets were also associated with a lower faecal scores during day 6-12 period compared to control-diet group (2.2 vs 3.2 ± 0.25). The casein hydrolysate and β glucan dietary treatment groups were not associated with any difference in fecal scores compared to control diet group during the overall experimental period.

The casein hydrolysate, β glucan and casein hydrolysate + β glucan dietary treatment groups were not associated with any difference in weights compared to control diet group of piglets. Only the ZnO group was associated with an increase in the weight of piglets at the end of experiment relative to the control-diet group (8.64 vs 7.49 ± 0.36 kg, P<0.05).

**Table 2: Effect of feed additives on faecal scores and weights of weaning piglets**

| Measurements | | Control | ZnO | casein hydrolysate | β glucan | casein hydrolysate + β glucan | SEM | Sign. |
|---|---|---|---|---|---|---|---|---|
| Faecal Score | Day 0-6 | 2.6 | 1.9 | 2.3 | 2.3 | 2.0 | 0.35 | ns |
| | Day 6-12 | | | | | | | ^{a} P<0.05, |
| | | 3.2 | 2.0^{b} | 3.0 | 3.4 | 2.2^{a} | 0.25 | ^{b} P<0.01 |
| | Overall | 2.8 | 1.9^{a} | 2.7 | 2.8 | 2.3 | 0.21 | ^{a} P<0.01 |
| Weight (kg) | Weight 0-6 | 7.19 | 7.53 | 7.28 | 7.35 | 7.66 | 0.18 | ns |
| | Weight 6-12 | 7.49 | 8.64^{a} | 7.63 | 7.47 | 8.13 | 0.36 | ^{a} P<0.05 |

| | | | | | | | | |
|---|---|---|---|---|---|---|---|---|
| ns: not significant, P>0.05 | | | | | | | | |

### Growth performance

The effect of feed additives like ZnO, casein hydrolysate, yeast β glucan and casein hydrolysate + β glucan on measurements of growth performance parameters over a period of 12 days is presented in Table 3. The ZnO and casein hydrolysate + β glucan dietary groups were associated with an increase in the overall average daily gain relative to control-diet group (0.10, 0.10 vs 0.00 ± 0.03 kg/day, P<0.05). The dietary groups ZnO, casein hydrolysate, β glucan and casein hydrolysate + β glucan were also associated with an increase in the overall feed intake in piglets relative to control-diet group (0.37, 0.34, 0.34, 0.36 vs 0.27 ± 0.02 kg/day, P<0.05). The dietary groups of ZnO and casein hydrolysate + β glucan were also associated with an increase in gain to feed ratio relative to control-diet group (0.26, 0.27 vs - 0.11 ± 0.11 kg/kg, P<0.05).

**Table 3: Effect of feed additives on overall average daily gain (OADG), overall feed intake (OFI) and gain to feed ratio in weaning piglets**

| Measurements | Control | ZnO | casein hydrolysate | β glucan | casein hydrolysate + β glucan | SEM | Significance |
|---|---|---|---|---|---|---|---|
| OADG (kg/day) (Day 0-12) | 0.00 | 0.10* | 0.02 | 0.00 | 0.10* | 0.03 | * P<0.05 |
| OFI (kg/day) (Day 0-12) | 0.27 | 0.37* | 0.34* | 0.34* | 0.36* | 0.02 | * P<0.05 |
| Gain to feed (kg/kg) (Day 0-12) | -0.10 | 0.26* | 0.05 | -0.03 | 0.27* | 0.11 | * P<0.05 |

### Microbiology

The effect of ZnO, casein hydrolysate, yeast β glucan and casein hydrolysate + β glucan dietary treatment groups on the abundance of a selected panel of bacterial groups present in caecal and colonic digesta is presented in Figure 1.

The casein hydrolysate dietary treatment group was associated with an increase in the abundance of total bacteria copy numbers compared to control-diet group (12.83 vs 12.40 ± 0.17 log₁₀ bacteria/ g DM digesta, P<0.05) in caecal digesta. The ZnO and casein hydrolysate dietary treatment groups were associated with an increase in abundance of *Bacteroidetes* copy numbers compared to control-diet group (12.60, 12.56 vs 11.71 ± 0.27 log₁₀ bacteria/ g DM digesta, P<0.05).

However, ZnO and β glucan treatment groups were also associated with a reduction in *Bifidobacteria spp.* numbers compared to control-diet group (8.94, 8.99 vs 9.37 ± 0.11 log₁₀ bacteria/ g DM digesta, P<0.05). The β glucan group was associated with an increase in *AEEC strains* compared to control-diet receiving group (10.49 vs 9.32 ± 0.22 log₁₀ bacteria/ g DM digesta, P<0.01). There was no effect on abundance of *Firmicutes, Lactobacilli* and *Enterobacteria* copy numbers observed in this experiment.

In colonic digesta, the ZnO, β glucan and casein hydrolysate + β glucan dietary treatment groups were associated with a reduction in abundance of *Bifidobacteria* copy numbers relative to control-diet group (8.96, 8.87, 8.95 vs 9.45 ± 0.17 log₁₀ bacteria/ g DM digesta, P<0.05). No change in bacterial copy numbers of any other bacterial group was associated with the dietary treatment groups of ZnO, casein hydrolysate, β glucan and casein hydrolysate + β glucan.

### Cytokine gene expression analysis

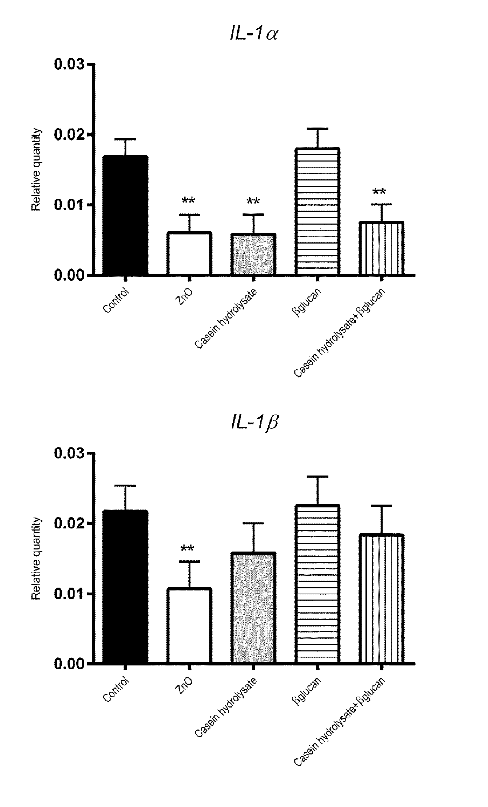

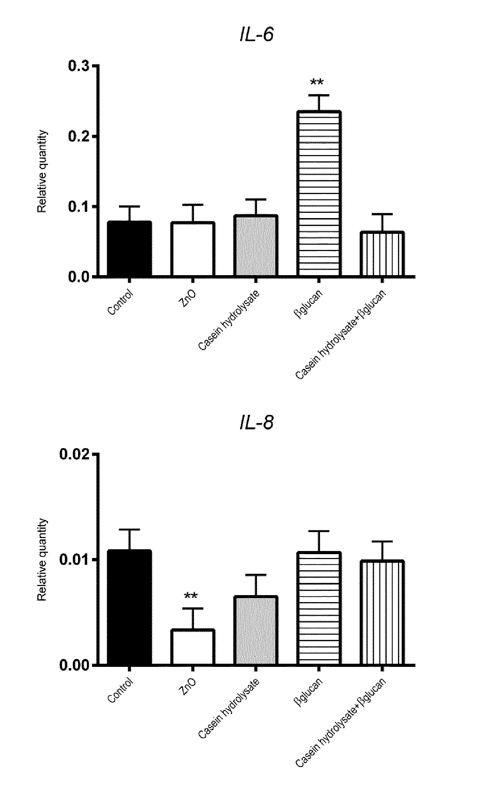

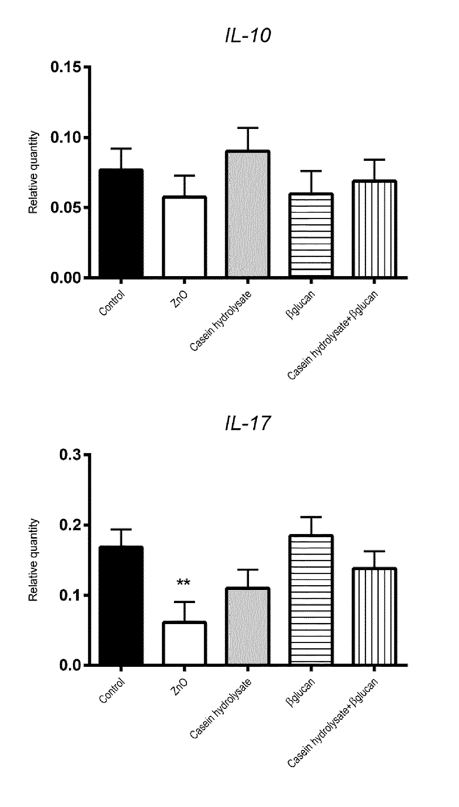

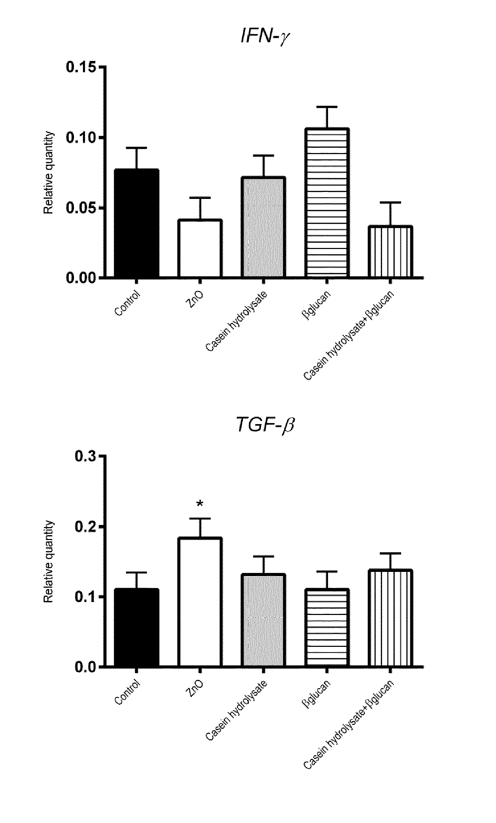

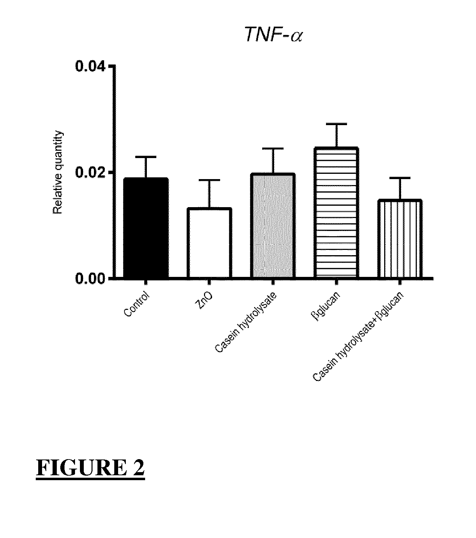

The effect of dietary treatment groups of ZnO, casein hydrolysate, yeast β glucan and casein hydrolysate +β glucan on expression of a selected panel of cytokine genes in colonic tissues is presented in Figure 2. The ZnO dietary treatment group was associated with a decrease in *IL-1 α* (0.006 vs 0.016 ± 0.002 RQ), *IL-1β* (0.010 vs 0.021 ± 0.003 RQ), *IL-8* (0.003 vs 0.010 ± 0.001 RQ) and *IL-17* (0.061 vs 0.168 ± 0.024 RQ) expression compared to control group. While, ZnO treatment group was also associated with an increase in *TGF-β* expression (0.183 vs 0.110 ± 0.022 RQ) compared to control group.

The casein hydrolysate group was associated with a decrease in *IL-1α* expression relative to control-diet group (0.005 vs 0.016 ± 0.002 RQ). The dietary β glucan treatment group was associated with an increase in *IL-6* expression relative to control diet group (0.235 vs 0.078 ± 0.025 RQ). The casein hydrolysate +β glucan treatment group was associated with a decrease in *IL-1α* (0.007 vs 0.016 ± 0.002 RQ) expression compared to control group. *IL-4, IL-21* and *FOXP3* expression was un-detectable in this study.

### References

Burrin, D. (2003). Intestinal nutrient requirements in weanling pigs.
Lalles, JP, Bosi, P, Smidt, H, & Stokes, CR. (2007). Nutritional management of gut health in pigs around weaning. [Review]. The Proceedings of the Nutrition Society, 66(2), 260-268.
Mukhopadhya, A, O'Doherty, JV, Smith, A, Bahar, B, & Sweeney, T. (2012). The microbiological and immunomodulatory effects of spray-dried versus wet dietary supplementation of seaweed extract in the pig gastrointestinal tract. Journal of Animal Science, 90 Suppl 4, 28-30.
Nataro, J, & Sussman, M. (2002). Diarrhoeagenic Escherichia coli. Molecular medical microbiology: Volumes 1-3, 1463-1504.
NRC. (1998). National Research Council, Nutrient Requirements of Swine, 10th Revised Edition.. National Academy Press, Washington DC., 111-141.
Ryan, MT, Smith, AG, O'Doherty, JV, Bahar, B, Reilly, P, & Sweeney, T. (2010). Effects of nutrient supplementation with laminarin derived from Laminaria hyperborea and Laminaria digitata on mucin gene expression in the porcine ileum. Livestock Science, 133(1-3), 236-238.
Sales, J. (2013). Effects of Pharmacological Concentrations of Dietary Zinc Oxide on Growth of Post-weaning Pigs: A Meta-analysis. Biological Trace Element Research, 1-7*.*
Sargeant, HR, McDowall, KJ, Miller, HM, & Shaw, MA. (2010). Dietary zinc oxide affects the expression of genes associated with inflammation: Transcriptome analysis in piglets challenged with ETEC K88. Veterinary Immunology and Immunopathology, 137(1-2), 120-129.
SAS. (2004). SAS users guide (Version 9.1.2). Cary, N.C: SAS Institue Inc.
Sweeney, T, Collins, CB, Reilly, P, Pierce, KM, Ryan, M, & O'Doherty, JV. (2012). Effect of purified beta-glucans derived from Laminaria digitata, Laminaria hyperborea and Saccharomyces cerevisiae on piglet performance, selected bacterial populations, volatile fatty acids and pro-inflammatory cytokines in the gastrointestinal tract of pigs. The British Journal of Nutrition, 108(7), 1226-1234.
Walsh, A, Sweeney, T, O'Shea, C, Doyle, D, & Doherty, J. (2013). Effect of supplementing varying inclusion levels of laminarin and fucoidan on growth performance, digestibility of diet components, selected faecal microbial populations and volatile fatty acid concentrations in weaned pigs. Animal Feed Science and Technology, In Press, Corrected Proof.

## Claims

1. A composition suitable for oral administration to a mammal comprising beta-glucan and casein hydrolysate, wherein the weight ratio of beta-glucan to casein hydrolysate is 1:2 to 2:1.

2. A composition as claimed in Claim 1 in which the casein hydrolysate is depleted in high molecular weight peptides.

3. An animal feed comprising a composition as claimed in Claims 1 or 2.

4. An animal feed as claimed in Claim 3 in which the composition comprises 0.01 to 0.03% of the animal feed by weight.

5. An animal feed as claimed in Claim 3 or 4 formulated for a weaning piglet, a weaning child, a weaning mammal, a pregnant or lactating mammal, a pregnant or lactating mother.

6. A composition of any of Claims 1 or 2, or an animal feed of any of Claims 3 to 5, for use in maintaining or improving gut health in a mammal.

7. A composition of any of Claims 1 or 2, or an animal feed of any of Claims 3 to 5, for use in preventing or treating a gastrointestinal inflammatory condition in a mammal.

8. A pharmaceutical composition comprising an effective amount of beta-glucan and casein hydrolysate in combination with a pharmaceutically acceptable excipient, wherein the weight ratio of beta-glucan to casein hydrolysate is 1:2 to 2:1.

9. A non-therapeutic method of improving growth performance of a weaning mammal comprising a step of administering to the weaning mammal or a maternal mammal an effective amount of beta-glucan and casein hydrolysate, wherein the weight ratio of beta-glucan to casein hydrolysate is 1:2 to 2:1.

10. A non-therapeutic method according to Claim 9 comprising administering to the weaning mammal 10-1000 mg beta-glucan per day per Kg body weight and 10-1000 mg casein hydrolysate per day per Kg body weight.

11. A non-therapeutic method according to Claim 10 comprising administering to the weaning mammal 10-100 mg beta-glucan per day per Kg body weight and 10-100 mg casein hydrolysate per day per Kg body weight.

12. A non-therapeutic method according to any of Claims 9 to 11 in which the beta-glucan and casein hydrolysate are administered to the weaning mammal during and after weaning.

13. A non-therapeutic method according to any of Claims 9 to 11 in which the beta-glucan and casein hydrolysate are administered to the maternal mammal during and after pregnancy.

14. A non-therapeutic method according to any of Claims 9 to 13 in which the casein hydrolysate is depleted in high molecular weight peptides.
